(19) Europäisches Patentamt
European Patent Office
Office européen des brevets

(11) Veröffentlichungsnummer: **0 137 176**
**B1**

(12) # EUROPÄISCHE PATENTSCHRIFT

(45) Veröffentlichungstag der Patentschrift: **17.02.88**

(51) Int. Cl.⁴: **C 07 D 405/06**, C 08 G 59/02

(21) Anmeldenummer: **84109114.3**

(22) Anmeldetag: **01.08.84**

(54) **N,N'-Bis-(2,3-epoxypropyl)-derivate cyclischer Dicarbonsäurehydrazide und ein Verfahren zu ihrer Herstellung.**

(30) Priorität: **12.08.83 DE 3329192**

(43) Veröffentlichungstag der Anmeldung: **17.04.85 Patentblatt 85/16**

(45) Bekanntmachung des Hinweises auf die Patenterteilung: **17.02.88 Patentblatt 88/7**

(84) Benannte Vertragsstaaten: **CH DE FR GB LI**

(56) Entgegenhaltungen:
**DD - A - 150 465**
**DE - A - 2 037 943**
**DE - A - 2 263 827**
**DE - A - 2 300 010**
**US - A - 3 692 705**

**CHEMICAL ABSTRACTS, vol. 98, no. 11, 14. März 1983, Columbus, Ohio, USA, G.V. SHATALOV, S.A. GRIDCHIN, G.V. KOVALEV, B.I. MIKHANT'EV, S.M. GOFMAN, "Allylation of compounds containing a pyridazine ring", Seite 552, abstract no. 89286s**

(73) Patentinhaber: **BAYER AG, Konzernverwaltung RP Patentabteilung, D-5090 Leverkusen 1 Bayerwerk (DE)**

(72) Erfinder: **Rottmaier, Ludwig, Ing. grad., Bergstrasse 85, D-5068 Odenthal-Glöbusch (DE)**
Erfinder: **Merten, Rudolf, Dr., Berta-von-Suttner-Strasse 55, D-5090 Leverkusen (DE)**

**EP 0 137 176 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N,N'-Bis-(2,3-epoxypropyl)-derivate cyclischer Dicarbonsäurehydrazide und ein Verfahren zu ihrer Herstellung.

Die Herstellung von O-substituierten Monoglycidylderivaten des Maleinsäurehydrazids ist aus der DD-PS 118 641 bekannt. Gemäss den Patentansprüchen 1 und 2 dieser Patentanschrift kann Maleinsäurehydrazid (falls in Formel XIV des Anspruchs 1 n für Null, $R^1$ und $R^4$ für H stehen) trotz des Überschusses von 2 bis 5 Mol Epichlorhydrin in Gegenwart von 1,3 bis 3 Mol Alkalihydroxid lediglich in das O-substituierte O-Monoepoxid überführt werden. Überraschenderweise konnten nun neue, teilweise radikalisch copolymerisierbare N,N'-Bis-(2,3-epoxypropyl)-derivate cyclischer Dicarbonsäurehydrazide hergestellt werden, sofern spezielle Verfahrensbedingungen eingehalten werden.

Aus der EP-A-0 044 422 ist bekannt, Glycidyl-1,2,4-Triazolidin-3,5-Dione als Imprägnier- und Anstrichmittel zu verwenden. Deren Eigenschaften konnten nochmals verbessert werden, wenn N,N-bis-(2,3-Oxipropyl)-Derivate cyclischer Dicarbonsäurehydrazide zur Herstellung dieser Mittel verwendet werden.

Gegenstand der Erfindung sind somit N,N'-Bis-(2,3-epoxypropyl)-derivate cyclischer Dicarbonsäurehydrazide der allgemeinen Formel (I),

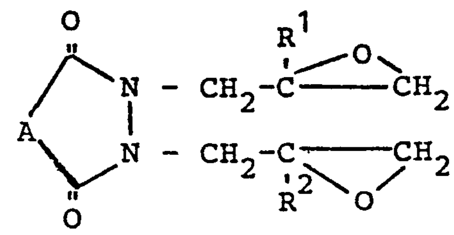
(I)

worin

$R^1$ und $R^2$ gleich oder verschieden ein Wasserstoffatom oder eine Methylgruppe, vorzugsweise ein Wasserstoffatom,

die Reste

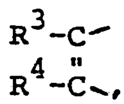,
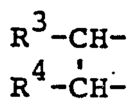,
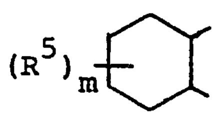,
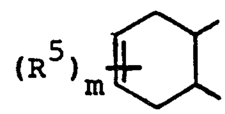,

(A¹) (A²) (A³) (A⁴)

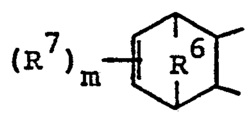,
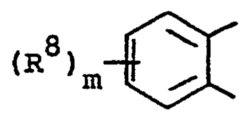

(A⁵) (A⁶)

vorzugsweise ($A^1$), ($A^4$), ($A^5$) und ($A^6$),

$R^3$ und $R^4$ gleich oder verschieden ein Wasserstoffatom, eine geradkettige oder verzweigte $C_1$–$C_4$-Alkylgruppe, Halogen oder eine $C_1$–$C_4$-Alkoxygruppe, vorzugsweise ein Wasserstoffatom oder eine Methylgruppe,

$R^5$ eine geradkettige $C_1$–$C_4$-Alkylgruppe oder Halogen, vorzugsweise eine Methylgruppe oder Chlor,

$R^6$ eine $CH_2$-Gruppe, eine $CCl_2$-Gruppe oder ein Sauerstoffatom, vorzugsweise eine $-CH_2$-Gruppe,

$R^7$ und $R^8$ Halogen, vorzugsweise Chlor und

m eine ganze Zahl von 0 bis 4, besonders bevorzugt die Zahl 0, bedeuten.

Besonders bevorzugt sind N,N'-Bis-(2,3-epoxypropyl)-derivate des Maleinsäure-, Citraconsäure-, Tetrahydrophthalsäure-, Phthalsäure- und Endomethylen-tetrahydrophthalsäurehydrazids.

Die erfindungsgemässen N,N'-Bis-(2,3-epoxypropyl)-derivate der cyclischen Dicarbonsäurehydrazide der allgemeinen Formel (I) werden erhalten, indem man cyclische Dicarbonsäurehydrazide der Formel (II)

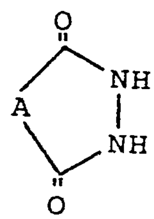
(II)

worin A die für Formel (I) angegebene Bedeutung hat, mit überschüssigem Epihalogenhydrin oder β-Methylhalogenhydrin wie z.B. Epichlorhydrin, Epibromhydrin oder β-Methylepichlorhydrin in Gegenwart eines geeigneten Katalysators zu den entsprechenden N,N'-Bis-(halogenhydrinen) dieser Hydrazide umsetzt, anschliessend entstehende Nebenprodukte durch Absaugen und/oder Auswaschen mit Wasser abtrennt und daran anschliessend durch Behandlung mit Halogenwasserstoffakzeptoren Halogenwasserstoff abspaltet.

Die zur Herstellung der neuen Glycidylverbindungen der allgemeinen Formel (I) verwendeten Hydrazide sind grösstenteils aus der Literatur bekannt bzw. können nach bekannten Verfahren, z.B. aus ungesättigten Anhydriden, Säuren, Estern und Hydrazin, wie z.B. in C.A. 78,147984 t oder in Journal of the American Chemical Society 80, 3790 (1958) beschrieben, hergestellt werden. Die gesättigten Hydrazide können aus den entsprechenden ungesättigten Hydraziden, wie z.B. Bernsteinsäurehydrazid aus Maleinsäurehydrazid in Journal of the American Chemical Society 73, 4716 (1951) beschrieben, erhalten werden.

Beispielhaft seien erwähnt: Maleinsäurehydrazid, Citraconsäurehydrazid, Bernsteinsäurehydrazid, Tetrahydrophthalsäurehydrazid, Methyltetrahydrophthalsäurehydrazid, Endomethylentetrahydrophthalsäurehydrazid, Methylendomethylentetrahydrophthalsäurehydrazid, Phthalylhydrazid, Tetrachlorphthalylhydrazid, Hexahydrophthalsäurehydrazid.

Beim erfindungsgemässen Verfahren werden in der ersten Stufe die cyclischen Hydrazide mit einem Epihalogenhydrin in Anwesenheit von geeigneten Katalysatoren zur Halogenhydrinverbindung der allgemeinen Formel (III)

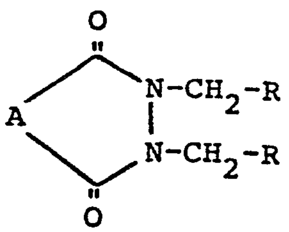

(III)

in der A die für Formel (I) angegebenen Reste und die Reste R in 1,2-Epoxyethylreste umwandelbare Reste bedeuten, umgesetzt.

Ein solcher, in einen 1,2-Epoxyethylrest umwandelbarer Rest R ist vor allem ein an verschiedenen Kohlenstoffatomen funktionelle Gruppen tragender Hydroxyhalogenethylrest wie die 2-Chlor-1-hydroxy- oder 2-Methyl-2-chlor-1-hydroxyethylreste.

Vorzugsweise verwendet man bei der Addition des Halogenhydrins als Katalysatoren quaternäre Ammoniumsalze wie Trimethylbenzylammoniumhydroxid, Tetraethylammoniumchlorid, Trimethylbenzylammoniumchlorid und Trimethylphenylammoniumchlorid.

Ausserdem sind noch Imidazoline wie 2-Phenylimidazolin, 2-Phenyl-4-methylimidazolin, 2-Methylimidazolin, 2,4-Dimethylimidazolin, 2-Ethylimidazolin, 2-Benzylimidazolin, 1,2-Phenylen-bis-imidazolin und Tetramethylen-bis-imidazolin geeignet.

Eine weitere Gruppe von geeigneten Katalysatoren sind cyclische Amidine der allgemeinen Formel (IV),

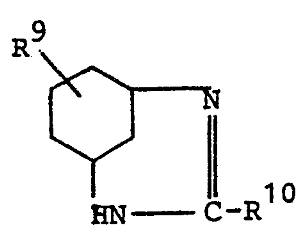

(IV)

worin

$R^9$ Wasserstoff oder eine Alkylgruppe mit 1 bis 4 C-Atomen

$R^{10}$ Wasserstoff oder einen organischen Rest, insbesondere Methyl, Ethyl, Propyl, 2-Ethylhexyl, Stearyl, Cyclohexyl, Phenyl und Benzyl

bedeuten. Die Herstellung dieser cyclischen Amidine ist in der DE-OS 3 041 834 beschrieben.

Ferner sind noch tertiäre Amine wie Triethylamin, Tri-n-butylamin, Triethanolamin, N,N′-Dimethylanilin, Benzyldimethylamin, Pyridin, Endoethylenpiperazin, N,N′-Dimethylpiperazin, 4-Dimethylaminomethyl-2,6-di-tert.-butylphenol und 2,4,6-Tris-dimethylaminomethyl-phenol geeignet.

Die Menge an Katalysator liegt vorzugsweise zwischen 0,01 und 10 Mol.-%, bezogen auf cyclisches Dicarbonsäurehydrazid.

Die Reaktion der cyclischen Carbonsäurehydrazide mit dem Epihalogenhydrin wird mit mindestens äquivalenten Mengen an Epihalogenhydrin durchgeführt, d.h., eine Hydrazidgruppe wird mit mindestens zwei Mol Epihalogenhydrin zur Reaktion gebracht. Vorzugsweise wird jedoch ein Überschuss an Epihalogenhydrin, und zwar 4 bis 60 Mol, besonders bevorzugt 10 bis 30 Mol Epihalogenhydrin pro Hydrazidgruppe verwendet. Aus wirtschaftlichen Gründen wird man die Menge an Epihalogenhydrin natürlich so niedrig wie möglich halten.

Die Reaktion zwischen den Hydraziden und Epihalogenhydrin kann bei 20 bis 160°C, vorzugsweise bei 30 bis 80°C, besonders bevorzugt bei 40 bis 60°C, gegebenenfalls unter erhöhtem Druck, durchgeführt werden. Bei der Verwendung von tertiären Aminen, insbesondere von stark basischen tertiären Aminen als Katalysator, sollten 60°C nicht wesentlich überschritten werden, da sonst Nebenprodukte in besonders hohem Masse gebildet werden, die die Bildung von N,N′-Diepoxiden bei der Dehydrohalogenierung stark zurückdrängen. Dieser Effekt wird weiter verstärkt, falls die Nebenprodukte vor der Dehydrohalogenierung nicht entfernt werden.

Die Reaktionszeiten liegen im allgemeinen zwischen 30 Minuten und mehreren Tagen, können in besonderen Fällen jedoch auch darüber oder darunter liegen. Durch entsprechende Wahl der Reaktionsbedingungen, z.B. Druck, werden kürzere Reaktionszeiten erreicht.

Die bei der Addition der Halogenhydrine an die Hydrazide entstehenden Nebenprodukte werden vor der 2. Stufe aus dem Reaktionsgemisch entfernt. Dies kann erfolgen durch Absaugen der auskristallisierten Verunreinigungen und/oder durch Auswaschen mit Wasser.

Anschliessend wird in einer zweiten Stufe die Halogenhydrinverbindung, in erster Linie das Bischlorhydrinhydrazid, das jedoch je nach Epihalogenhydrin- oder β-Methylhalogenhydrinüberschuss bereits gewisse Mengen an Glycidylverbindungen enthalten kann, mittels Halogenwasserstoff-abspaltender Verbindungen zu den N,N′-Bis-(2,3-epoxypropyl)-derivaten der cycli-

schen Dicarbonsäurehydrazide dehydrohalogeniert.

Die zur Halogenwasserstoffabspaltung eingesetzten alkalisch reagierenden Verbindungen sind insbesondere Alkali- oder Erdalkalimetallhydroxide wie Natriumhydroxid, Kaliumhydroxid, Calciumhydroxid, vorzugsweise Natriumhydroxid. Dies kann in fester Form oder in Lösung, vorzugsweise in 20–50%igen wässrigen Lösungen, eingesetzt werden.

Ferner können zur Halogenwasserstoffabspaltung Alkalicarbonate, insbesondere Soda und Kaliumcarbonat in fester oder gelöster Form, Alkalisilikate, Alkaliphosphate und Alkalialuminate sowie überschüssiges Epihalogenhydrin oder 1,2-Alkylenoxide, wie Ethylenoxid, Verwendung finden, wobei bei Verwendung von Epichlorhydrin dieses in Glycerindichlorhydrin umgewandelt wird.

Auf eine Halogenhydringruppe der Verbindungen der Formel (III) werden 1 bis 1,2 Äquivalente der Halogenwasserstoff abspaltenden Verbindungen eingesetzt.

Zweckmässig soll bei der Halogenwasserstoffabspaltung der pH-Wert nicht über 13, vorzugsweise nicht über pH 11 ansteigen. Um dies zu erreichen, setzt man das Alkali nach und nach zu bzw. tropft die Lösung allmählich zu und kontrolliert dabei den pH-Wert der Reaktionsmischung.

Die Halogenwasserstoffabspaltung kann im Temperaturbereich von 20°C bis 120°C erfolgen. Wird die Halogenwasserstoffabspaltung mit Alkali, z.B. Kalilauge, durchgeführt, so ist zur Erzielung optimaler Ausbeuten eine Reaktionstemperatur von 70°C nicht zu überschreiten. Die besten Ergebnisse werden bei Temperaturen um 25 bis 35°C erhalten. Bei Einsatz von Alkalicarbonaten sollte die Reaktionstemperatur jedoch über 70°C liegen, wobei die Höchstgrenze in der Regel durch die Siedetemperatur des überschüssigen Epihalogenhydrins vorgegeben ist.

Bei der Dehydrohalogenierung ist es vorteilhaft, ein mit Wasser nicht mischbares organisches Lösungsmittel zuzusetzen, um das bei der Reaktion entstehende bzw. das über die Alkalilösung zugetropfte Wasser azeotrop entfernen zu können. Die Mengen an zugesetztem Lösungsmittel sind nicht kritisch. Geeignete Lösungsmittel sind chlorierte Kohlenwasserstoffe wie Methylenchlorid, Chloroform, Ethylenchlorid oder Trichlorethylen. Sofern für die Bildung der Bis-(chlorhydrin)-Verbindungen der cyclischen Dicarbonsäurehydrazide ein grosser Überschuss an Epihalogenhydrin eingesetzt wurde, kann das überschüssige Epihalogenhydrin als mit Wasser nicht mischbares Lösungsmittel fungieren.

Eine besonders bevorzugte Ausführungsform besteht darin, dass man Hydrazide der Formel (II) mit Epihalogenhydrin, vorzugsweise Epichlorhydrin, in Gegenwart eines geeigneten Katalysators, vorzugsweise einer quaternären Ammoniumbase, eines quaternären Ammoniumsalzes, eines Imidazolins oder eines cyclischen Amidins, in einer ersten Stufe umsetzt, entstehende Nebenprodukte durch Absaugen oder Auswaschen mit Wasser entfernt, und das dabei entstandene Halogenhydrin-Gruppen enthaltende Hydrazid in einer zweiten Stufe mit halogenwasserstoffabspaltenden Verbindungen, insbesondere Alkali-, Erdalkalihydroxiden oder Alkalicarbonaten in fester oder gelöster Form behandelt.

Die Aufarbeitung der Bis-(2,3-epoxypropyl)-dicarbonsäurehydrazide erfolgt in der Regel so, dass das bei der Halogenwasserstoffabspaltung entstehende Nebenprodukt, z.B. Kochsalz bei Verwendung von Natronlauge als Säurefänger, durch Absaugen entfernt wird. Eventuell noch vorhandene Kochsalz- und Alkalireste werden durch Auswaschen mit Wasser entfernt.

Selbstverständlich können jedoch auch das gesamte Kochsalz und eventuell vorhandene Alkalireste ohne vorheriges Absaugen durch Auswaschen mit Wasser entfernt werden. Die verbleibende Lösung wird dann gegebenenfalls nach dem Trocknen über einem geeigneten Trocknungsmittel wie wasserfreiem Natriumsulfat, vom Lösungsmittel, z.B. überschüssigem Epichlorhydrin, welches in den nächsten Ansätzen wieder mitverwendet werden kann, gegebenenfalls im Vakuum entfernt, und die erhaltenen hellgelb bis gelb gefärbten viskosen Öle werden durch Auflösen in geeigneten Lösungsmitteln wie $C_1$–$C_4$-Alkanolen, vorzugsweise Methanol, Ethanol, Ketonen wie Butanon, Glykol- bzw. Diglykolmonoether oder deren Acetate wie Ethylen-glykolmonomethylether, Diethylen-glykolmonoethylether und Ethylenglykolmonoethyletheracetat, Essigsäureester wie Essigsäureethylester oder -butylester, aromatische Kohlenwasserstoffe wie Toluol oder Xylol bzw. deren Gemische und nachfolgendem Abkühlen zur Kristallisation gebracht.

Die erhaltenen kristallinen Verbindungen können abgesaugt und gegebenenfalls durch Umkristallisieren, z.B. aus Ethanol oder Essigsäurebutylester, weiter gereinigt werden. Häufig kann jedoch auf eine Reinigung verzichtet und das Rohprodukt sofort weiterverarbeitet werden.

Je nach Konstitution des Restes A entstehen häufig viskose Harze, die dann ohne weitere Reinigung weiter umgesetzt werden können.

Die erfindungsgemäss erhaltenen Glycidyl-hydrazide besitzen Epoxidwerte von 0,3 bis 0,893, vorzugsweise 0,5 bis 0,893. Unter Epoxidwert wird die Anzahl von Epoxidäquivalenten verstanden, die in 100 g Substanz enthalten ist.

Das Epoxidäquivalent ist definiert als die Grammenge Substanz, in der eine 1,2-Epoxidgruppe enthalten ist.

Eine 1,2-Epoxidgruppe ist einem Mol Halogenwasserstoff äquivalent.

Die erfindungsgemäss hergestellten Polyglycidylverbindungen können allein oder zusammen mit üblichen Härtungsmitteln, als Imprägnierungsmittel für Textilien, z.B. Fasern aus Polyester, als Überzugsmittel, z.B. für Anstrichzwekke auf Glas, Metallen und Holz, als Klebemittel für Kunststoffe verschiedenster Art, z.B. zum Verkleben von ungewebten textilartigen Gebilden, sowie zur Herstellung von Formkörpern, z.B. Giess-, Presskörpern und Laminaten verwendet werden.

Die nachfolgenden Beispiele sollen die Erfindung näher erläutern, wobei, wenn nicht anders angegeben, die Prozente Gewichtsprozente und Teile Gewichtsprozente bedeuten.

Beispiel 1

112 g Maleinsäurehydrazid, 1200 g Epichlorhydrin und 2 g Tetraethylammoniumchlorid werden 20 Stunden bei 60°C gerührt. Nach dem Abkühlen wird von ungelösten Bestandteilen abgesaugt, und zur Epichlorhydrinlösung werden bei 30°C 180 g einer 45%igen Natronlauge getropft. Zur Vervollständigung der Reaktion wird 4 Stunden bei 30°C nachgerührt. Das schmierig angefallene Kochsalz kann nach Zugabe von 250 g Wasser ausgewaschen werden. Die organische Phase wird nochmals mit 100 g Wasser gewaschen, über Natriumsulfat getrocknet und im Vakuum bei 50°C und 0,3 mbar eingeengt. Es werden 124 g eines hellbraunen Epoxidharzes erhalten, welches über Nacht durchkristallisiert und welches laut gaschromatographischer Analyse (= GC) im wesentlichen aus N,N'-Bis-(2,3-epoxypropyl)-maleinsäurehydrazid (Reinheitsgrad 93,8%) besteht. Durch Umkristallisieren aus Essigsäurebutylester kann das reine N,N'-Bis-(2,3-epoxypropyl)-maleinsäurehydrazid vom Fp. = 58°C erhalten werden, dessen Struktur mittels IR- und NMR-Spektren sowie der Elementaranalyse bestätigt wird.

Ber.: für $C_{10}H_{12}N_2O_4$ (224,2):
53,57% C; 5,40% H; 12,50% N
Gef.: 53,7% C; 5,5% H; 12,4% N.

Beispiel 2

112 g Maleinsäurehydrazid, 1200 g Epichlorhydrin und 2 g des Amidins der Formel IV mit $R^9$ = Methyl und $R^{10}$ = Phenyl werden 34 Stunden bei 60°C gerührt und wie in Beispiel 1 weiterbearbeitet. Es werden 113 g eines bräunlichen Harzes erhalten, welches laut GC im wesentlichen aus N,N'-Bis-(2,3-epoxypropyl)-maleinsäurehydrazid (Reinheitsgrad 94,1%) besteht.

Beispiel 3

112 g Maleinsäurehydrazid, 1200 g Epichlorhydrin und 2 g Tetraethylammoniumchlorid werden 3 Stunden bei 80°C gerührt, wobei zwischenzeitlich wegen der einsetzenden exothermen Reaktion gekühlt werden muss. Die noch warme Lösung wird mit 200 g Wasser ausgeschüttelt und bei 30°C mit 80 g Ätznatron portionsweise versetzt. Zur Vervollständigung der Reaktion wird 4 Stunden bei 30°C nachgerührt und das entstandene Kochsalz abgesaugt. Nach dem Auswaschen der Epichlorhydrinlösung mit 100 g Wasser wird diese mit $Na_2SO_4$ getrocknet, filtriert und bei 50°C/0,3 mbar eingeengt. Es resultieren 107 g eines viskosen Öles, welches allmählich erstarrt und welches laut GC im wesentlichen aus N,N'-Bis-(2,3-epoxypropyl)-maleinsäurehydrazid besteht.

Beispiel 4

63 g Citraconsäurehydrazid, 800 g Epichlorhydrin und 1 g Tetraethylammoniumchlorid werden 12 Stunden bei 60°C gerührt und mit 100 g Wasser ausgeschüttelt. Dann werden bei einer Sumpftemperatur von ca. 35°C 100 g einer 40%igen Natronlauge unter azeotropem Rückfluss zugetropft, dass das zugetropfte und das bei der Reaktion entstehende Wasser abgezogen werden kann. Zur Vervollständigung der Reaktion wird 2 Stunden unter azeotropem Rückfluss nachgerührt und das Kochsalz abgesaugt. Die Epichlorhydrinlösung wird 2mal mit 150 g Wasser ausgeschüttelt, über Natriumsulfat getrocknet und bei 50°C/0,3 mbar eingeengt. Es resultieren 138 g eines bräunlichen viskosen Öles mit einem Epoxidwert von 0,74 und einem Chlorgehalt von 1,2%, welches laut NMR-Spektrum hauptsächlich aus dem N,N'-Bis-(2,3-epoxypropyl)-citraconsäurehydrazid (GC: 89,9%ig) besteht.

Beispiel 5

81 g Phthalsäurehydrazid, 1000 g Epichlorhydrin und 1 g Tetraethylammoniumchlorid werden 4 Stunden bei 80°C gerührt und nach dem Abkühlen abgesaugt. Die Epichlorhydrinlösung wird bei 30°C mit 100 g einer 40%igen Natronlauge versetzt, 4 Stunden bei 30°C gerührt, sodann mit 300 g Wasser und schliesslich mit 100 g Wasser ausgewaschen. Nach dem Trocknen der organischen Phasen und Einengen bei 60°C und 0,3 mbar werden 82 g eines viskosen, gelblichen Harzes mit einem Epoxidwert von 0,59 und einem Chlorgehalt von 2,3% erhalten, aus dem durch Kristallisation mit einem Ethanol/Isopropanol-Gemisch das reine N,N'-Diglycidylphthalsäurehydrazid vom Fp = 102°C erhalten wird. IR- und NMR-Spektren sowie die Elementaranalyse bestätigen die angenommene Struktur.

Ber.: für $C_{14}H_{14}N_2O_4$ (274,3):
61,30% C; 5,14% H; 10,21% N,
Gef.: 61,2% C; 5,2% H; 10,3% N.

Beispiel 6

112 g Maleinsäureanhydrid, 1200 g Epichlorhydrin und 10 g Tetraethylammoniumchlorid werden 20 Stunden bei 40°C gerührt und wie in Beispiel 1 weiterverarbeitet. Es wurden 109 g eines gelben Harzes erhalten, welches laut GC-Analyse im wesentlichen aus N,N'-Bis-(2,3-epoxypropyl)-maleinsäurehydrazid (Reinheitsgrad 92,9%) besteht.

Beispiel 7

112 g Maleinsäurehydrazid, 1200 g Epichlorhydrin und 1,5 g Triethylamin werden 16 Stunden bei 60°C gerührt. Unlösliche Bestandteile werden durch Absaugen entfernt. Nach dem Dehydrohalogenieren mit 180 g 45%iger Natronlauge wird wie in Beispiel 1 aufgearbeitet. Es werden 119 g eines bräunlichen Epoxidharzes erhalten, welches laut GC-Analyse aus N,N'Bis-(2,3-epoxypropyl)-maleinsäurehydrazid mit einem Reinheitsgrad von 95,8% besteht.

Beispiel 8

41,5 g Tetrahydrophthalsäurehydrazid, 500 g Epichlorhydrin und 0,5 g Tetraethylammoniumchlorid werden 25 Stunden bei 80°C gerührt und nach dem Abkühlen mit 100 g Wasser ausgewaschen. Zur Epichlorhydrinlösung werden bei 30°C 50 g einer 40%igen Natronlauge getropft und zur Vervollständigung der Reaktion 7 Stunden bei 30°C nachgerührt. Das Kochsalz wird durch zweimaliges Auswaschen mit 200 g Wasser entfernt. Nach dem Trocknen über Natriumsulfat und Einengen bei 60°C und 0,3 mbar werden 36,5 g eines hellgelben, viskosen Epoxidharzes mit einem Epoxidwert von 0,62 und einem Chlorgehalt von 2,1% erhalten, das im wesentlichen aus N,N'-Bis-(2,3-epoxypropyl)-tetrahydrophthalsäurehydrazid besteht.

Anwendungsbeispiel

100 g N,N'-Diglycidylphthalsäurehydrazid und 112 g Hexahydrophthalsäureanhydrid werden getrennt aufgeschmolzen, bei 110°C vermischt und in eine Form gegossen. Nach der Härtung von 4 Stunden bei 80°C und 16 Stunden bei 160°C werden Prüfkörper hergestellt, die folgende mechanische Eigenschaften aufweisen:

| | |
|---|---|
| Biegefestigkeit | nach DIN 53452 in N/mm² 158 |
| Durchbiegung | nach DIN 53452 in N/mm² 3,8 |
| Schlagzähigkeit | nach DIN 53453 in KJ/m² 9,5 |
| Kugeldruckhärte | nach DIN 53456 in N/mm² 180 |
| Martensgrad | nach DIN 53458 in °C 167 |

Vergleichsbeispiel 1

Dieses Beispiel zeigt, dass ohne Abtrennen der bei der Umsetzung der Hydrazide mit Epihalogenhydrin in Gegenwart von tertiären Amionen entstehenden Nebenprodukte nach der Dehydrohalogenierung ein Epoxidgruppen enthaltendes Reaktionsprodukt erhalten wird, das N,N'-Bis-(2,3-epoxypropyl)-dicarbonsäurehydrazid in nur geringen Mengen enthält.

112 g Maleinsäurehydrazid, 1200 g Epichlorhydrin und 1,5 g Triethylamin werden 16 Stunden bei 60°C gerührt. Nach dem Abkühlen wird ohne Abtrennen der Nebenprodukte sofort mit 180 g 45%iger Natronlauge wie in Beispiel 7 weitergearbeitet. Es werden 117 g eines bräunlichen Epoxidharzes mit einem Epoxidwert von 0,65 erhalten, das auch nach 4 Wochen Lagerung nicht kristallisierte. Laut GC-Analyse enthält das Harz 27,1% N,N'-Bis-(2,3-epoxypropyl)-maleinsäurehydrazid.

Vergleichsbeispiel 2

Dieser Vergleich zeigt, dass bei höheren Temperaturen als 60°C und tertiären Aminen als Katalysatoren und ohne Abtrennen der Nebenprodukte nach der Umsetzung der Hydrazide mit Epihalogenhydrin die Ausbeuten an N,N'-Diepoxiden weiter verringert werden.

112 g Maleinsäurehydrazid, 1200 g Epichlorhydrin und 1,5 g Triethylamin werden 45 Minuten bei Rückflusstemperatur gerührt. Nach dem Abkühlen wird mit 180 g 45%iger Natronlauge wie in Beispiel 7 weitergearbeitet. Es werden 109 g eines schwarzbraunen, nicht kristallisierenden Harzes erhalten, welches laut GC-Analyse 19,2% N,N'-Bis-(2,3-epoxypropyl)-maleinsäurehydrazid enthält.

Vergleichsbeispiel 3

Dieser Versuch zeigt, dass bei der direkten Umsetzung der Carbonsäurehydrazide mit Epihalogenhydrin in Gegenwart von starken Basen kein N,N'-Bis-(2,3-epoxypropyl)-dicarbonsäurehydrazid erhalten wird.

Analog dem Beispiel 1 der DD-PS 118 641 werden 22,4 g Maleinsäurehydrazid (60,2 Mol), 22,4 g Kaliumhydroxid, 20 g Wasser, 74 g (60,8 Mol) Epichlorhydrin (entspricht 2 Mol pro NH-Gruppe) und 250 ml Ethanol 4 Stunden bei 70°C gerührt, bis kein Maleinsäurehydrazid mehr nachzuweisen ist. Der entstandene Niederschlag von Kaliumchlorid wird abgesaugt und das Filtrat bei vermindertem Druck (ca. 20 mbar) bei 40°C 4 Stunden eingeengt. Es werden 46,5 g (Theorie 44,8) eines hellgelben viskosen Öls erhalten, das auch nach 4wöchigem Stehen (selbst nach Verrühren mit Lösungsmitteln wie Methanol, Aceton, Essigsäureethylester und Essigsäurebutylester nicht kristallisiert. Der Epoxidwert des erhaltenen Öls direkt nach der Herstellung betrug 0,33 (theoretischer Epoxidwert für ein Diepoxid 0,89).

Bei der dünnschichtchromatographischen Analyse (Laufmittel 80 Vol-% Chloroform und 20 Vol-% Methanol) konnte kein N,N'-(Bis-(2,3-epoxypropyl)-maleinsäurehydrazid nachgewiesen werden.

**Patentansprüche**

1. N,N'-Bis-(2,3-epoxypropyl)-derivate cyclischer Dicarbonsäurehydrazide der Formel (I)

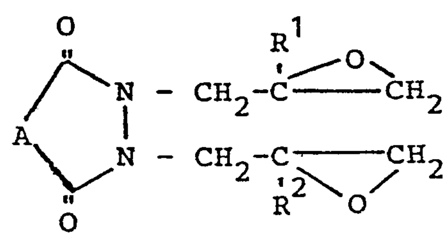

(I)

worin

$R^1$ und $R^2$ gleich oder verschieden ein Wasserstoffatom oder eine Methylgruppe,

A die Reste

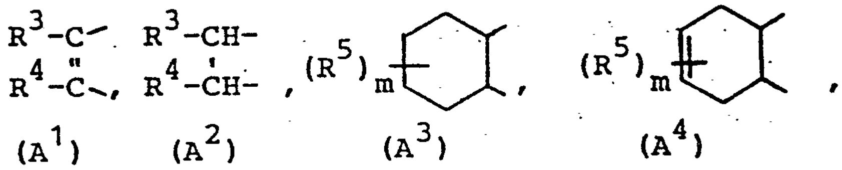

(A¹) (A²) (A³) (A⁴)

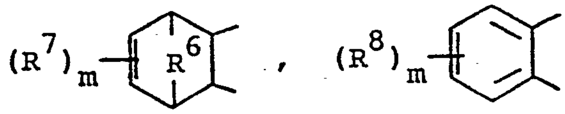

(A⁵) (A⁶)

$R^3$ und $R^4$ gleich oder verschieden ein Wasserstoffatom, eine geradkettige oder verzweigte $C_1$–$C_4$-Alkylgruppe, Halogen oder eine $C_1$–$C_4$-Alkoxygruppe,
$R^5$ eine geradkettige $C_1$–$C_4$-Alkylgruppe oder Halogen,
$R^6$ eine $-CH_2-$, $-CCl_2$-Gruppe oder ein Sauerstoffatom,
$R^7$ und $R^8$ Halogen und
m eine ganze Zahl von 0 bis 4 bedeuten.

2. N,N′-Bis-(2,3-epoxypropyl)-derivate cyclischer Dicarbonsäurehydrazide gemäss Anspruch 1, dadurch gekennzeichnet, dass in Formel (I)

$R^1$ und $R^2$ ein Wasserstoffatom,
A die Reste ($A^1$), ($A^4$), ($A^5$) und ($A^6$),
$R^3$ und $R^4$ gleich oder verschieden ein Wasserstoffatom oder eine Methylgruppe,
$R^5$ eine Methylgruppe oder Chlor,
$R^6$ eine $-CH_2$-Gruppe,
$R^7$ und $R^8$ Chlor und
m die Zahlen 0 bis 4 bedeuten.

3. N,N′-Bis-(2,3-epoxypropyl)-derivate cyclischer Dicarbonsäurehydrazide gemäss Anspruch 1, dadurch gekennzeichnet, dass in Formel (I)

$R^1$ und $R^2$ ein Wasserstoffatom,
A die Reste ($A^1$) und ($A^6$),
$R^3$ und $R^4$ gleich oder verschieden ein Wasserstoffatom oder eine Methylgruppe, und
m 0 bedeuten.

4. Verfahren zur Herstellung von Glycidylhydraziden gemäss Anspruch 1, dadurch gekennzeichnet, dass cyclische Dicarbonsäurehydrazide der Formel (II),

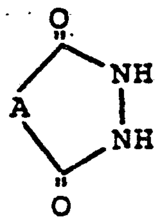

(II)

worin
A die in Anspruch 1 angegebene Bedeutung hat, mit mindestens der äquivalenten Menge an Epihalogenhydrin oder β-Methylhalogenhydrin in Gegenwart eines geeigneten Katalysators, zu den entsprechenden N,N′-Bis-(halogenhydrinen) der cyclischen Dicarbonsäurehydrazide umgesetzt, entstehende Nebenprodukte durch Absaugen und/oder Auswaschen mit Wasser abgetrennt und anschliessend mit Halogenwasserstoffakzeptoren Halogenwasserstoff abgespalten werden.

**Claims**

1. N,N′-bis-(2,3-epoxypropyl) derivatives of cyclic dicarboxylic acid hydrazides of the formula (I)

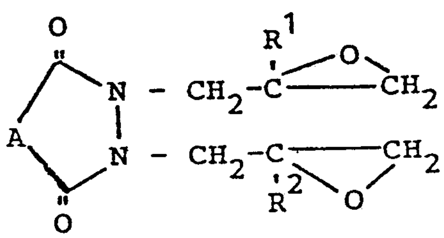

(I)

wherein
$R^1$ and $R^2$ are identical or different and denote a hydrogen atom or a methyl group,
A denotes the radicals

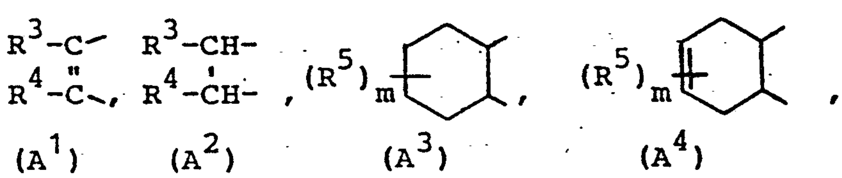

(A¹) (A²) (A³) (A⁴)

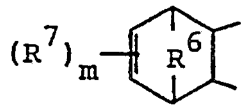

($A^5$)

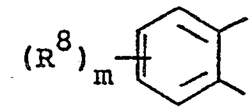

($A^6$)

$R^3$ and $R^4$ are identical or different and denote a hydrogen atom, a straight-chain or branched $C_1$–$C_4$-alkyl group, halogen or a $C_1$–$C_4$-alkoxy group,
$R^5$ denotes a straight-chain $C_1$–$C_4$-alkyl group or halogen,
$R^6$ denotes a $-CH_2-$, $-CCl_2$ group or an oxygen atom,
$R^7$ and $R^8$ denote halogen and
m denotes an integer from 0 to 4.

2. N,N′-bis-(2,3-epoxypropyl) derivatives of cyclic dicarboxylic acid hydrazides according to Claim 1, characterised in that in formula (I)
$R^1$ and $R^2$ denote a hydrogen atom,
A denotes the radicals ($A^1$), ($A^4$), ($A^5$) and ($A^6$),
$R^3$ and $R^4$ are identical or different and denote a hydrogen atom or a methyl group,
$R^5$ denotes a methyl group or chlorine,
$R^6$ denotes a $-CH_2$ group,
$R^7$ and $R^8$ denote chlorine and
m denotes the numbers 0 to 4.

3. N,N′-bis-(2,3-epoxypropyl) derivatives of cyclic dicarboxylic acid hydrazides according to Claim 1, characterised in that in formula (I)
$R^1$ and $R^2$ denote a hydrogen atom,
A denotes the radicals ($A^1$) and ($A^6$),
$R^3$ and $R^4$ are identical or different and denote a hydrogen atom or a methyl group, and
m denotes 0.

4. Process for the preparation of glycidyl hydrazides according to Claim 1, characterised in that cyclic dicarboxylic acid hydrazides of the formula (II)

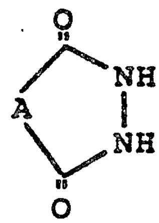

(II)

wherein
A has the meaning given in Claim 1,
are reacted with at least the equivalent quantity of epihalohydrin or β-methylhalohydrin in the presence of a suitable catalyst, to give the corresponding N,N′-bis-(halohydrins) of the cyclic dicarboxylic acid hydrazides, any by-products formed are separated off by drawing them off by suction and/or by washing them out with water and hydrogen halide is then split off using hydrogen halide acceptors.

**Revendications**

1. Dérivés N,N′-bis-(2,3-époxypropyliques) d'hydrazides d'acides dicarboxyliques cycliques de formule (I)

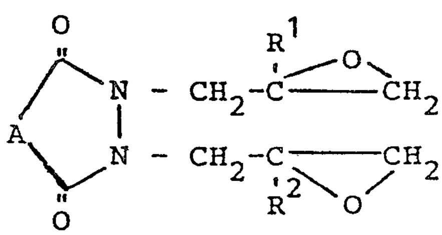

(I)

dans laquelle
$R^1$ et $R^2$ sont identiques ou différents et représentent un atome d'hydrogène ou un groupe méthyle,
A représente les restes

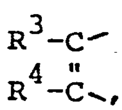

($A^1$)

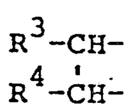

($A^2$)

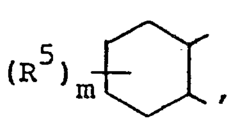

($A^3$)

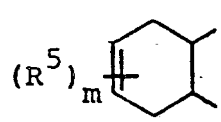

($A^4$)

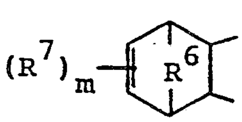

($A^5$)

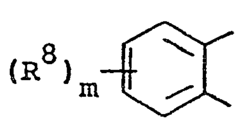

($A^6$)

$R^3$ et $R^4$ sont identiques ou différents et représentent un atome d'hydrogène, un groupe alkyle en $C_1$ à $C_4$ à chaîne droite ou à chaîne ramifiée, un halogène ou un groupe alkoxy en $C_1$ à $C_4$,
$R^5$ est un groupe alkyle en $C_1$ à $C_4$ à chaîne droite ou un halogène,
$R^6$ est un groupe $-CH_2$, $-CCl_2$ ou un atome d'oxygène,
$R^7$ et $R^8$ représentent un halogène et
m est un nombre entier de 0 à 4.

2. Dérivés N,N'-bis-(2,3-époxypropyliques) d'hydrazides d'acides dicarboxyliques cycliques suivant la revendication 1, caractérisés en ce que dans la formule (I)
$R^1$ et $R^2$ représentent un atome d'hydrogène,
A représente les restes ($A^1$), ($A^4$), ($A^5$) et ($A^6$),
$R^3$ et $R^4$, identiques ou différents, représentent un atome d'hydrogène ou un groupe méthyle,
$R^5$ est un groupe méthyle ou du chlore,
$R^6$ est un groupe $-CH_2$,
$R^7$ et $R^8$ représentent du chlore et
m représente les nombres de 0 à 4.

3. Dérivés N,N'-bis-(2,3-époxypropyliques) d'hydrazides d'acides dicarboxyliques cycliques suivant la revendication 1, caractérisés en ce que, dans la formule (I)
$R^1$ et $R^2$ représentent un atome d'hydrogène,
A représente les restes ($A^1$) et ($A^6$),
$R^3$ et $R^4$, identiques ou différents, représentent un atome d'hydrogène ou un groupe méthyle, et
m est égal à 0.

4. Procédé de production de glycidylhydrazides suivant la revendication 1, caractérisé en ce qu'on fait réagir des hydrazides d'acides dicarboxyliques cycliques de formule (II)

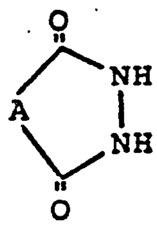

(II)

dans laquelle
A a la définition indiquée dans la revendication 1, avec au moins la quantité équivalente d'épihalogénhydrine ou de β-méthylhalogénhydrine en présence d'un catalyseur approprié, pour former les N,N'-bis-(halogénhydrines) correspondantes des hydrazides d'acides dicarboxyliques cycliques, on sépare les sous-produits formés par filtration par succion et/ou par extraction par lavage à l'eau, puis on élimine l'halogénure d'hydrogène avec des accepteurs d'halogénure d'hydrogène.